(19) Europäisches Patentamt
European Patent Office
Office européen des brevets

(11) Veröffentlichungsnummer: **0 007 490**
**B1**

(12) # EUROPÄISCHE PATENTSCHRIFT

(45) Veröffentlichungstag der Patentschrift: **24.02.82**

(21) Anmeldenummer: **79102292.4**

(22) Anmeldetag: **05.07.79**

(51) Int. Cl.³: **C 07 C 177/00,**
A 61 K 31/557
//C07D319/14, C07D407/12

(54) **Neue Prostaglandinderivate der Delta-2,4-11-Desoxy-PGE-Reihe, Verfahren zu ihrer Herstellung sowie ihre Verwendung als Arzneimittel.**

(30) Priorität: **08.07.78 DE 2830079**

(43) Veröffentlichungstag der Anmeldung:
**06.02.80 Patentblatt 80/3**

(45) Bekanntmachung des Hinweises auf die Patenterteilung:
**24.02.82 Patentblatt 82/8**

(84) Benannte Vertragsstaaten:
**AT BE CH DE FR GB IT NL SE**

(56) Entgegenhaltungen:
**KEINE**

(73) Patentinhaber: **HOECHST AKTIENGESELLSCHAFT**
**Zentrale Patentabteilung Postfach 80 03 20**
**D-6230 Frankfurt/Main 80 (DE)**

(72) Erfinder: **Bartmann, Wilhelm, Dr.**
**Am Dachsbau 5**
**D-6232 Bad Soden am Taunus (DE)**
Erfinder: **Beck, Gerhard, Dr.**
**Gustav-Freytag-Strasse 24**
**D-6000 Frankfurt am Main (DE)**
Erfinder: **Lerch, Ulrich, Dr.**
**Schwalbenweg 19**
**D-6238 Hofheim am Taunus (DE)**
Erfinder: **Bickel, Martin, Dr.**
**Breitlacherstrasse 25**
**D-6000 Frankfurt am Main 90 (DE)**

EP 0 007 490 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Neue Prostaglandinderivate der Δ2,4-11-Desoxy-PGE-Reihe, Verfahren zu ihrer Herstellung sowie ihre Verwendung als Arzneimittel

Prostaglandine sine eine Gruppe von Fettsäuren, die in zahlreichen Geweben und Organen von Mensch und Tier vorkommen. Das Grundgerüst der natürlichen Prostglandine besteht aus 20 Kohlenstoffatomen, die in Form eines Fünfrings und zweier benachbarter linearer Seitenketten angeordnet sind (zur Nomenklatur siehe: N. Andersen, Annals of the New York Academy of Sciences, Vol. 180, Prostaglandins, S. 14).

Die pharmakologischen Effekte der Prostaglandine erstrecken sich u.a. auf die Gebiete der Reproduktion, des Bronchialmuskeltonus, des Blutdrucks und der Gastroenterologie. Die pharmakologischen Eigenschaften der natürlichen Prostaglandine sind Gegenstand zahlreicher Übersichtsartikel, z.B. N.H. Andersens und P.W. Ramwell in Arch. Internal Med. *133*, 30(1974); R.L. Jones in Pathobiology Ann. 1972; J. Pike in Scient. American *225*, 84 (1971) oder M.P.L. Caton in Progress in Med. Chem. Vol. 8, ed.: Butterworth, London 1971.

Die Synthese von nicht natürlich vorkommenden Analogen von Prostansäuren, in denen die Vielzahl der pharmakologischen Wirkungen der natürlichen Prostaglandine differenziert sind, gewinnt zunehmend an Bedeutung.

Die vorliegende Erfindung betrifft neue Verbindungen der Formel I

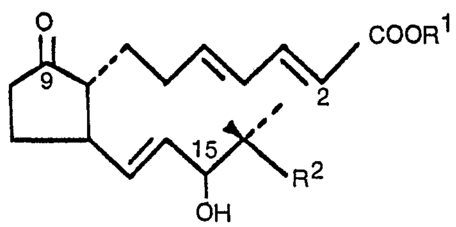

I

die strukturell mit den natürlichen Prostaglandinen verwandt sind und in welcher bedeuten

$R^1$ a) Wasserstoff oder einen geradkettigen, verzweigten, gesättigten oder ungesättigten aliphatischen oder cycloaliphatischen Kohlenwasserstoff-Rest mit bis zu zehn Kohlenstoffatomen,

b) ein physiologisch verträgliches Metall- oder $NH_4$-Ion oder ein Ammoniumion, das sich von einem primären, sekundären oder tertiären Amin ableitet,

$R^2$ einen geradkettigen, aliphatischen Kohlenwasserstoff-Rest mit einem bis sechs Kohlenstoffatomen.

Bevorzugt beeinhaltet die vorliegende Erfindung Verbindungen der Formel I, worin bedeuten

$R^1$ a) Wasserstoff oder einen geradkettigen oder verzweigten Alkylrest mit bis zu sechs Kohlenstoffatomen oder einen cycloaliphatischen Kohlenwasserstoff-Rest mit drei bis sieben Kohlenstoffatomen,

b) ein physiologisch verträgliches Metall- oder $NH_4$-Ion oder ein Ammoniumion, das sich von einem primären, sekundären oder tertiären Amin ableitet,

$R^2$ einen geradkettigen, aliphatischen Kohlenwasserstoff-Rest mit zwei bis sechs Kohlenstoffatomen.

Unter den Substituenten $R^1$ sind die im folgenden aufgeführten besonders bevorzugt:

Wasserstoff, ein geradkettiger oder verzweigter Alkylrest mit bis zu sechs Kohlenstoffatomen, insbesondere Methyl, Äthyl, n-Propyl, n-Butyl, n-Pentyl, n-Hexyl, Isopropyl und tert.-Butyl sowie Cyclobutyl, Cyclopentyl, Cyclohexyl und Cycloheptyl.

Gegenstand der Erdindung ist ferner ein Verfahren zur Herstellung von Verbindungen der formel I, das dadurch gekennzeichnet ist, daß man

a) einen Aldehyd der Formel II

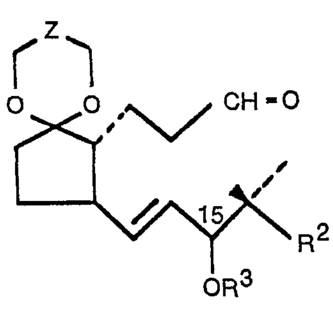

II

worin Z eine $CH_2$-Gruppe, eine —$C(CH_3)_2$-Gruppe oder eine Einfachbindung bedeutet, $R^2$ die in Formel I angegebene Bedeutung hat und $R^3$ eine leicht abspaltbare Schutzgruppe darstellt, umsetzt mit einem Ylid der Formel III

$$(R^4)_3P{=}CH{-}CH{=}CH{-}COOR^1 \qquad III$$

worin $R^1$ die zur Formel I genannte Bedeutung hat, jedoch kein Kation darstellt, und worin die Substituenten $R^4$ gleich oder verschieden sein können und geradkettiges ($C_1$—$C_4$)Alkyl oder Phenyl bedeuten,

b) den erhaltenen Ester der Formel IV

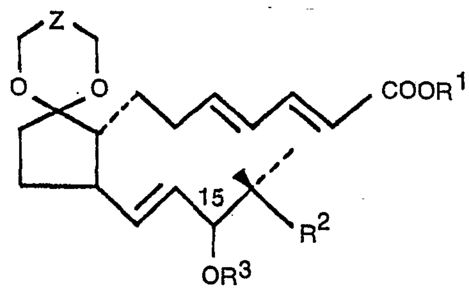

IV

worin $R^1$ die in Formel III, $R^2$ die zur Formel I in Anspruch 1 und $R^3$ und Z die in Formel II angegebenen Bedeutungen haben, durch saure Hydrolyse überführt in eine Verbindung der Formel I,
gegebenenfalls die erhaltene Verbindung der Formel I, worin $R^1$ die zur Formel III und $R^2$ die zur Formel I in Anspruch 1 angegebene Bedeutung haben, durch Behandlung mit Basen in eine Verbindung der Formel I, worin $R^1$ Wasserstoff bedeutet und $R^2$ die Zur Formel I in Anspruch 1 angegebene Bedeutung hat, überführt,
gegebenenfalls eine Verbindung der Formel I, worin $R^2$ die zur Formel I in Anspruch 1 angegebene Bedeutung hat und $R^1$ Wasserstoff bedeutet, zu einer Verbindung der Formel I, worin $R^1$ und $R^2$ die zur Formel I in Anspruch 1 angegebenen Bedeutungen haben, wobei $R^1$ jedoch kein kation bedeutet, verestert
und gegebenenfalls eine Verbindung der Formel I, worin $R^2$ die zur Formel I in Anspruch 1 angegebene Bedeutung hat und $R^1$ Wasserstoff bedeutet, in ein physiologisch verträgliches Metall- oder Aminsalz überführt.

Die Verfahrensschritte zur Herstellung von Verbindungen de Formel I ($R^1$=H) können durchgeführt werden in Analogie zu dem in der DT—OS 2 546 313 (HOE 75/F 270) beschriebenen Verfahren. Die Veresterung von Verbindungen der Formel I, die als freie Säure vorliegen, erfolgt nach Methoden, wie sie in der DT—OS 2 628 564 (HOE 76/F 149) beschrieben sind.

Die Ausgangsstoffe der Formel II können gemäß der DT—OS 2 407 186 hergestellt werden.

Die erfindungsgemäßen Verbindungen der Formel I werden gewöhnlich in Form ihrer Racemate erhalten. Diese können gegebenenfalls nach den üblichen Methoden der Racemattrennung in Form der optisch aktiven Antipoden erhalten werden.

Falls keine Epimerentrennung auf der Stufe der Alkohole der allgemeinen Formel IV erfolgt ist, kann man mit den Verbindungen der allgemeinen Formel I eine Epimerentrennung der 15-ständigen Alkohole durchführen.

Außer den in den Beispielen genannten lassen sich insbesondere auch die folgenden Verbindungen der Formel I herstellen:

15-Hydroxy-9-oxo-16,16-dimethyl-(E)-2,(E)-4,(E)-13-prostatriensäure-äthylester
15-Hydroxy-9-oxo-16,16-dimethyl-(E)-2,(E)-4,(E)-13-prostatriensäure-isopropylester
15-Hydroxy-9-oxo-16,16-dimethyl-18,19,20-,trinor-(E)-2,(E)-4,(E)-13-prostatriensäure
15-Hydroxy-9-oxo-16,16-dimethyl-18,19,20-trinor-(E)-2,(E)-4,(E)-13-prostatriensäure-methylester
15-Hydroxy-9-oxo-16,16-dimethyl-(E)-2,(E)-4,(E)-13-prostatriensäure-cyclohexylester
15-Hydroxy-9-oxo-16,16-dimethyl-(E)-2,(E)-4,(E)-13-prostatriensäure-äthylester
15-Hydroxy-9-oxo-16,16-dimethyl-20-nor-(E)-2,(E)-4,(E)-13-prostatriensäure-äthylester.

Die erfindungsgemäßen Verbindungen zeichnen sich insbesondere durch magensäuresekretionshemmende und ulcusprotektive Eigenschaften aus, wie durch Versuche an der Ratte festgestellt werden konnte. So hemmt die Verbindung gemäß Beispiel 6a nach oraler Gabe von 0,03—1,0 mg/kg dosisabhängig sowohl die Ulcusentwicklung als auch die Magensäuresekretion an der Ratte. Im Enteropoolingtest (erhöhtes intestinales Flüssigkeitsvolumen nach PG—Gabe) an der Ratte (Lit.: Robert, A et. al Prostaglandins 11, (1976) Nr. 5, 809) zeichnen sich die erfindungsgemäßen Verbindungen durch einen günstigen Quotienten aus protektiver Wirkdosis und beginnender stimulatorischer Dosis (Enteropooling) aus.

Die erfindungsgemäßen Verbindungen der Formel I können als freie Säuren, in Form ihrer physiologisch unbedenklichen anorganischen und organischen Salze oder als Ester zur Anwendung kommen.

Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertige Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. Polyäthylenglykole oder auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten, sowie örtlich anwendbare Zubereitungen wie Cremes, Emulsionen, Suppositorien, insbesondere Aerosole in Frage kommen.

Eine weitere Anwendung der neuen Verbindungen liegt in der Kombination mit anderen Wirkstoffen.

Die erfindungsgemäßen Verbindungen wirken am Menschen mit einer oralen Tagesdosis von 0,1—20 mg/kg, bevorzugt 0,4—4 mg/kg magensäuresekretionshemmendund ulcusprotektiv. Als Doseinheit kommt für dieselben Indikationen ein Bereich von 2 mg—400 mg, bevorzugt von 8 mg—80 mg in Betracht.

Die Verbindungen der Formel IV sind neue wertvolle Zwischenprodukte für die Herstellung von Verbindungen der Formel I.

Beispiel 1

3-Carbomethoxy-2-propenyl-triphenylphosphoniumbromid

(0,425 Mol) 111,2 g Triphenylphosphin (Mol 262) in 480 ml Benzol abs. gelöst; unter kräftigem Rühren bei RT

(0,40 Mol) 71,6 g $\gamma$-Brom-crotonsäuremethylester innerhalb 1 1/2 Std. zugetropft; Temperatur steigt auf + 26°C; weißer Niederschlag. 24 Std. bei RT nachgerührt, Kristalle abgesaugt und 1 × mit Benzol und 2—3 mal mit Petroläther nachgewaschen. Im Trockenschrank bei 60°C unter Vakuum getrocknet.

Ausbeute: 139,9 g Fp.: roh 175°C (Zersetzungsprodukt) $[C_{23}H_{22}O_2P]$ Br (441,2)

| | | | | |
|---|---|---|---|---|
| Ber. | C 62,6 | H 5,03 | P 7,02 | Br 18,11 |
| Gef. | C 62,65 | H 5,2 | P 7,2 | Br 18,0 |

Beispiel 2

[3-Carbomethoxy-propen-(2)-yliden-(1)]-triphenyl-phosphoran III

(0,15 Mol) 66,15 g 3-Carbomethoxy-2-propenyl-triphenylphosphoniumbromid wurden in 3,5 l kaltem $H_2O$ gelöst. Danach wurden

(0,15 Mol) 6 g NaOH in 300 ml $H_2O$ gelöst und während 1 1/2 Std. zugetropft → gelb-organge Niederschlag. Danach wurde sofort abgesaugt und der Niederschlag gut mit $H_2O$ nachgewaschen. Das Produkt wurde auf einen Tonteller gegeben und im Exsikkator über KOH unter Vakuum 2 Tage vorgetrocknet, danach unter $P_2O_5$ vollständig getrocknet. Das Produkt wird unter Vakuum mit $P_2O_5$ im Exsikkator aufbewahrt.

Ausbeute: 39,5 g (III) Fp.: 166°C (sintern ab 160°C)

NMR: ($CDCl_3$) $\delta = 3,6$ (s, 3) $COOCH_3$; 7,1—8,0 (m, 16) $C_6H_5$—, —CH=P 5,2 (d, 1) *CH*=, 3,65 (d, 1) CH=*CH*—$COOCH_3$

Beispiel 3a

3[7(4,4-Dimethyl-3-tetrahydro-pyranyloxy-(E)-1-octen-1-yl)-1,4-dioxaspiro[4,4]-non-6-yl]-propionaldehyd II

($Z = CH_2$, $R^2 = n\text{-}C_4H_9$, $R^3 =$ 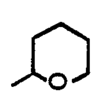 )

dargestellt analog Beispiel 14a—14d des in DT—OS 2 407 186 beschriebenen Verfahrens.

Gelbes Öl, das nicht weiter gereinigt wurde. Im UR besitzt die Verbindung II eine starke Carbonylbande bei

1730 $cm^{-1}$

D.C. $R_f$-Wert 0,53 auf Kieselgelplatten der Fa. Merck in Cyclohexan/Essigester 1:1

NMR ($CDCl_3$): $\delta$0,9 (d, c) $C(CH_3)_2$; 0,9 (t,3)—$CH_3$; 1,1—1,4(6)*$CH_2$* (THP); 1,4—2,7 (m, 16) *$CH_2$*—, *CH*; 3,2—4,0 (m, 3) *$CH_2$*O, *CH*—O—THP; 3,9 (s, 4) *$CH_2$*O; 4,5—4,7 (m, 2) —O—*CH*—O—; 5,2—5,6 (m, 2) CH=CH, 9,65 (s, 1) CH=O

Beispiel 3b

3[7(4,4-Dimethyl-3-tetrahydropyranyloxy-(E)-1-nonen-1-yl)-1,4-dioxaspiro[4,4]-non-6-yl]-propionaldehyd II

($Z = CH_2$, $r^2 = n\text{-}C_5H_{11}$, $R^3 =$ 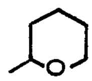 )

dargestellt analog Beispiel 3a.

NMR ($CDCl_3$): $\delta$0,9 (d, 6) $C(CH_3)_2$; 0,9 (t, 3) $CH_3$; 1,1—1,4 (6) *$CH_2$* (THP); 1,4—2,65 (m, 18) *$CH_2$*—, *CH*; 3,2—4,0 (m, 3) *$CH_2$*O, *CH*—O—THP; 3,9 (s, 4) *$CH_2$*O); 4,5—4,7 (m, 2) —O—*CH*—O—; 5,2—5,6 (m, 2) CH=CH, 9,6 (s, 1) CH=O

Beispiel 3c

3[7(4,4-Dimethyl-3-tetrahydropyranyloxy-(E)-1-hepten-1-yl)-1,4-dioxaspiro[4,4]-non-6-yl]-propionaldehyd II

($Z = CH_2$, $R^2 = n\text{-}C_3H_7$, $R^3 =$ 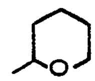 )

dargestellt analog Beispiel 3a.

NMR ($CDCl_3$): $\delta$ 0,9 (d, 6) $C(CH_3)_2$; 0,9 (t, 3) $CH_3$; 1,1—1,45 (6) *$CH_2$* (THP); 1,35—2,6 (m, 14) *$CH_2$*—, *CH*; 3,2—4,05 (m, 3) *$CH_2$*O, *CH*—O—THP; 3,95 (s, 4) *$CH_2$*O; 4,5—4,65 (m, 2) —O—*CH*—O; 5,1—5,7 (m, 2) CH=CH, 9,5 (s, 1) CH=O

Beispiel 4a

7-[7-(4,4-Dimethyl-3-tetrahydropyranyloxy-(E)-1-octen-1-yl)-1,4-dioxaspiro[4,4]-non-6-yl]-(E)-2,(E)-4,-heptadiensäure-methylester IV

($Z = CH_2$, $R^1 = CH_3$, $R^2 = n\text{-}C_4H_9$, $R^3 =$ 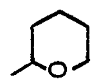 )

5,77 g (0,0136 Mol) 3[7(4,4 - Dimethyl - 3 - tetrahydropyranyloxy - (E) - 1 - octen - 1 - yl) - 1,4 - dioxaspiro[4,4] - non - 6 - yl] - pripionaldehyd (Beispiel 3a) werden in 120 ml abs. Benzol gelöst (Argon als Schutzgas). Zu dieser Lösung tropft man unter Feuchtigkeitsausschluß 14,74 g (0,0408 Mol) [3 - Carbomethoxy - propen - (2) - yliden - (1)] - triphenyl - phosphoran (Beispiel 2) und erwärmt anschließend 2 1/2 Stunden unter Rückfluß. Dann wird der Ansatz im Vakuum eingeengt, der Rückstand mit Äther extrahiert, die ätherischen Extrakte im Vakuum erneut eingeengt. Zurück bleibt ein gelbes Öl, welches an Kieselgel mit Äther als Elutionsmittel chromatograhiert wird.

Ausbeute: 6,21 g hellgelbes Öl (IV)

NMR ($CDCl_3$): $\delta$ 0,9 (d, 6) $C(CH_3)_2$; 0,9 (t, 3) $CH_3$; 1,1—1,4 (6) *$CH_2$* (THP); 1,4—2,7 (m, 16) *$CH_2$*, *CH*; 3,7 (s, 3) COO*$CH_3$*, 3,9 (s, 4) *$CH_2$*O 3,1—4,0 (m, 3) *$CH_2$*O, *CH*—OTHP; 4,5—4,65 (m, 2) O—CH—O; 5,0—6,8 (m, 6) olefin Protonen.

Beispiel 4b

7[7-(4,4-Dimethyl-3-tetrahydropyranyloxy-(E)-1-nonen-1-yl)-1,4-dioxaspiro[4,4]-non-6-yl]-(E)-2,(E)-4,-heptadiensäure-methylester IV

($Z = CH_2$, $R^1 = CH_3$, $R^2 = n\text{-}C_5H_{11}$, $R^3 =$ 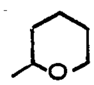 )

Reaktion analog beispiel 4 a aus 3[7(4,4 - Dimethyl - 3 - tetrahydropyranyloxy - (E) - 1 - nonen - 1 - yl) - 1,4 - dioxaspiro[4,4] - non - 6 - yl] - propionaldehyd

NMR ($CDCl_3$): $\delta$0,9 (d, 6) $C(CH_3)_2$; 0,9 (t, 3) $CH_3$; 1,1—1,4 (6) *$CH_2$* (THP); 1,4—2,65 (m, 18) *$CH_2$*, *CH*; 3,75 (s, 3) $COOCH_3$; 3,9 (s, 4) *$CH_2$*O; 3,1—4,0 (m, 3) *$CH_2$*O, *CH*—OTHP; 4,5—4,65 (m, 2) O—CH—O; 5,0—6,65 (m, 6) olefin. Protonen.

Beispiel 4c

7[7-(4,4-Dimethyl-3-tetrahydropyranyloxy-(E)-1-hepten-1-yl)-1,4-dioxaspiro[4,4]-non-6-yl]-(E)-2,(E)-4-heptadiensäure-methylester IV

($Z = CH_2$, $R^1 = Ch_3$, $R^2 = n\text{-}C_3H_7$, $R^3 =$ 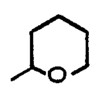 )

Reaktion analog Beispiel 4 a aus 3[7(4,4 - Dimethyl - 3 - tetrahydropyranyloxy - (E) - 1 - hepten - 1 - yl) - 1,4 - dioxaspiro[4,4] - non - 6 - yl] - propionaldehyd

NMR ($CDCl_3$): $\delta$ 0,9 (d, 6) $C(CH_3)_2$; 0,9 (t, 3) $CH_3$; 1,1—1,4 (6) *$CH_2$*(THP); 1,4—2,6 (m, 14) *$CH_2$*, *CH*; 3,75 (s, 3) $COOCH_3$; 3,9 (s, 4) $CH_2$O; 3,1—4,1 (m, 3) *$CH_2$*O, *CH*—O—THP; 4,5—4,65 (m, 2) O—CH—O; 5,1—6,7 (m, 6) olefin. Protonen

Beispiel 5a

7-[5-Oxo-2-(3-hydroxy-4,4-dimethyl-(E)-1-octen-1-yl)-cyclopentan-1-yl]-(E)-2,(E)-4-heptadiensäure-methylester I

($R^1 = CH_3$, $R^2 = n\text{-}C_4H_9$)

6,12 g 7[7 - (4,4 - Dimethyl - 3 - tetrahydropyranyloxy - (E) - 1 - octen - 1 - yl) - 1,4 - dioxaspiro[4,4] - non - 6 - yl] - (E) - 2,(E) - 4 - heptadiensäure - methylester (Beispiel 4a) werden in 200 ml Methanol gelöst. Zu dieser Lösung gibt man 30 ml kalt gesättigte, wässrige Oxalsäurelösung und rührt 2 Tage bei Raumtemperatur. Das Lösungsmittel wird dann i. Vakuum entfernt, der Rückstand mit gesättigter Natriumchloridlösung versetzt und dann mit Äther extrahiert. Es wird mit Wasser neutral gewaschen, mit $MgSO_4$ die Ätherphase getrocknet, i. Vakuum eingeengt.

Ausbeute: 4,4 g gelbes Öl
DC: $R_f = 0,43$ 15β-Epimeres
$R_f = 0,36$ 15α-Epimeres
auf Kieselgelplatten der Fa. Merck in Cyclohexan/Essigester = 1:1

Beispiel 5b

7[5-Oxo-2(3-hydroxy-4,4-dimethyl-(E)-1-nonen-1-yl)-cyclopentan-1-yl]-(E)-2,(E)-4-heptadiensäuremethylester I

$(R^1 = CH_3, R^2 = n\text{-}C_5H_{11})$

Reaktion analog Beispiel 5a aus 7[7(4,4 - Dimethyl - 3 - tetrahydropyranyloxy - (E) - 1 - nonen - 1 - yl) - 1,4 - dioxaspiro[4,4] - non - 6 - yl] - (E) - 2,(E) - 4 - heptadiensäuremethylester.

DC: $R_f = 0,42$ 15β-Epimeres
$R_f = 0,37$ 15α-Epimeres
auf Kieselgelplatten der Fa. Merck, in Cyclohexan/Essigester = 1:1

Beispiel 5c

7[5-Oxo-2(3-hydroxy-4,4-dimethyl-(E)-1-hepten-1-yl)-cyclopentan-1-yl]-(E)-2,(E)-4-heptadiensäuremethylester I

$(R^1 = CH_3, R^2 = n\text{-}C_3H_7)$

Reaktion analog Beispiel 5a aus 7[7(4,4 - Dimethyl - 3 - tetrahydropyranyloxy - (E) - 1 - hepten - 1 - yl) - 1,4 - dioxaspiro[4,4] - non - 6 - yl] - (E) - 2,(E) - 4 - heptadiensäuremethylester.

DC: $R_f = 0,44$ 15β-Epimeres
$R_f = 0,38$ 15α-Epimeres
auf Kieselgelplatten der Fa. Merck in Cyclohexan/Essigester = 1:1)

Beispiel 6a

7[5-Oxo-2-(3-hydroxy-4,4-dimethyl-(E)-1-octen-1-yl)-cyclopentan-1-yl]-(E)-2,(E)-4-heptadiensäure I

$(R^1 = H, R^2 = n\text{-}C_4H_9)$

4,4 g 7[5 - Oxo - 2(3 - hydroxy - 4,4 - dimethyl - (E) - 1 - octen - 1 - yl) - cyclopentan - 1 - yl] - (E) - 2,(E) - 4 - heptadiensäure - methylester werden in 10 ml Methanol gelöst. Zu dieser Lösung gibt man 23,4 ml 1 M NaOH (doppelter Überschuß) und rührt 48 Std. bei Raumtemperatur.- Reaktionsverlauf im Dünnschichtchromatogramm verfolgen-Danach wird 30 g Eis und festes Natriumchlorid zugegeben und mit 2 N Salzsäure auf pH 1—2 angesäuert. Die wässerige Phase wird mit Äther extrahiert, der Äther dann mit Wasser neutral gewaschen, getrocknet mit $MgSO_4$ eingeengt i. Vakuum der Rückstand an Kieselgel chromatographiert. Elutionsmittel Cyclohexan/Essigester/Eisessig = 40/60/1

Ausbeute: 3,2 g gelbes Öl
DC: $R_f = 0,35$ 15β-Epimeres
$R_f = 0,31$ 15α-Epimeres
(Kieselgelplatten der Fa. Merck in Cyclohexan/Essigester/Eisessig 40/60/1)

NMR ($CDCl_3$): δ 0,9 (s, 6) $C(CH_3)_2$; 1,1—2,8 (m, 16) —$CH_2$—, —CH—; 3,25—3,4 (m, 1) —*CH*~OH; 3,6—4,6 (breites Signal, 2) COO*H*, O*H*; 5,5—6,3 (m, 6) olefinische Protonen

Beispiel 6b

7[5-Oxo-2(3-hydroxy-4,4-dimethyl-(E)-1-nonen-1-yl)-cyclopentan-1-yl]-(E)-2,(E)-4-heptadiensäure I

$(R^1 = H, R^2 = n\text{-}C_5H_{11})$

Reaktion analog Beispiel 5a aus 7[5 - Oxo - 2(3 - hydroxy - 4,4 - dimethyl - (E) - 1 - nonen - 1 - yl) - cyclopentan - 1 - yl] - (E) - 2,(E) - 4 - heptadiensäuremethylester

DC: $R_f = 0,34$ 15β-Epimeres
$R_f = 0,30$ 15α-Epimeres

Kieselgelplatten der Fa. Merck in Cyclohexan/Essigester/Eisessig 40/60/1

NMR ($CDCl_3$): δ 0,9 (s, 6) $C(CH_3)_2$; 1,1—2,7 (m, 18) —$CH_2$, —CH—; 3,2—3,4 (m, 1) —C*H*—OH; 3,2—4,2 (breites Signal, 2) COO*H*, O*H*; 5,5—6,2 (m, 6) olefinische Protonen

Beispiel 6c

7[5-Oxo-2(3-hydroxy-4,4-dimethyl-(E)-1-hepten-1-yl)-cyclopentan-1-yl]-(E)-2,(E)-4-heptadiensäure I

($R^1 = H$, $R^2 = n\text{-}C_3H_7$)

Reaktion analog Beispiel 5a aus 7[5 - Oxo - 2(3 - hydroxy - 4,4 - dimethyl - (E) - 1 - hepten - 1 - yl) - cyclopentan - 1 - yl] - (E) - 2,(E) - 4 - heptadiensäuremethylester

DC: $R_f = 0,36$ 15β-Epimeres

$R_f = 0,32$ 15α-Epimeres

Kieselgelplatten der Fa. Merck in Cyclohexan/Essigester/Eisessig 40/60/1

NMR ($CDCl_3$): δ 0,9 (s, 6) $C(CH_3)_2$; 1,1—2,9 (m, 14) —$CH_2$, —CH—; 3,2—3,4 (m, 1) —CH~OH; 3,4—4,6 (breites Signal, 2) COOH, OH; 5,5—6,2 (m, 6) olefinische Protonen.

Beispiel 7a

Natriumsalz der 7[5-Oxo-2(3-hydroxy-4,4-dimethyl-(E)-1-octen-1-yl)-cyclopentan-1-yl]-(E)-2,(E)-4-heptadiensäure I

($R^1 = Na$, $R^2 = n\text{-}C_4H_9$)

362,5 mg 7[5 - Oxo - 2(3 - hydroxy - 4,4 - dimethyl - (E) - 1 - octen - 1 - yl) - cyclopentan - 1 - yl] - (E) - 2,(E) - 4 - heptadiensäure wird in 4 ml Athanol gelöst und 5 ml einer 0,2 molaren Lösung von Natriumhydroxyd in Äthanol zugegeben. Durch Verdampfen des Lösungsmittels erhält man das Natriumsalz als zähes Öl.

Beispiel 7b

Tromethaminsalz von 7[5-Oxo-2(3-hydroxy-4,4-dimethyl-(E)-1-nonen-1-yl)-cyclopenten-1-yl]-(E)-2,(E)-4-heptadiensäure I

($R^1 = H_3N^{\oplus}\text{—}C(CH_2OH_3$, $R^2 = n\text{-}C_5H_{11}$)

Eine Lösung von 188,25 mg 7[5 - Oxo - 2(3 - hydroxy - 4,4 - dimethyl - (E) - 1 - nonen - 1 - yl) - cyclopenten - 1 - yl] - (E) - 2,(E) - 4 - heptadiensäure in 4 ml Äthanol wird mit einer äthanolischer Lösung von 60,6 mg Tromethamin versetzt und das Lösungsmittel abgedampft, zuletzt im Hochvakuum.

Beispiel 8

7[5-Oxo-2(3-hydroxy-4,4-dimethyl-(E)-1-heptan-1-yl)-cyclopentan-1-yl]-(E)-2,(E)-4-heptadiensäure-äthylester I

($R^1 = C_2H_5$, $R^2 = n\text{-}C_3H_7$)

Eine Lösung von 350,5 mg 7[5 - Oxo - 2(3 - hydroxy - 4,4 - dimethyl - (E) - 1 - hepten - 1 - yl) - cyclopentan - 1 - yl] - (E) - 2,(E) - 4 - heptadiensäure in 6 ml Äther wird unter Eiskühlung mit 5 ml einer 1 M Diazoäthanlösung in Äther versetzt. Man läßt noch 30 Min. rühren und verdampft das Lösungsmittel mit dem überschüssigen Diazoäthan im Vakuum. Das Produkt ist chromatographisch rein.

NMR ($CDCl_3$): 1,25 (t, 3) —$COOCH_2CH_3$; 4,25 (q, 2) —$COOCH_2CH_3$ 5,5—6,2 (m, 6) olefinische Protonen

**Patentansprüche**

1. Verbindungen der Formel I

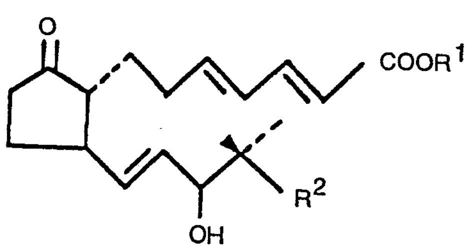

I

worin bedeuten

$R^1$ a) Wasserstoff oder einen geradkettigen, verzweigten, gesättigten oder ungesättigten aliphatischen oder cycloaliphatischen Kohlenwasserstoff-Rest mit bis zu 10 Kohlenstoffatomen, oder

b) ein physiologisch verträgliches Metall- oder $NH_4$-Ion oder ein Ammoniumion, das sich von einem primären, sekundären oder tertiären Amin ableitet,

$R^2$ einen geradkettigen, aliphatischen Kohlenwasserstoff-Rest mit einem bis sechs Kohlenstoffatomen bedeutet.

2. Verfahren zur Herstellung von Verbindungen der Formel I dadurch gekennzeichnet, daß man

a) einen Aldehyd der Formel II

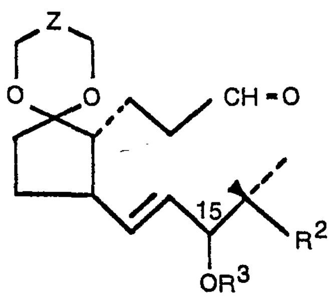

II

worin Z eine $CH_2$-Gruppe, eine $-C(CH_3)_2$-Gruppe oder eine Einfachbindung bedeutet, $R^2$ die in Formel I angegebene Bedeutung hat und $R^3$ eine leicht abspaltbare Schutzgruppe darstellt, umsetzt mit einem Ylid der Formel III

$$(R^4)_3P{=}CH{-}CH{=}CH{-}COOR^1 \qquad III$$

worin $R^1$ die zur Formel I genannte Bedeutung hat, jedoch kein Kation darstellt, und worin die Substituenten $R^4$ gleich oder verschieden sein können und geradkettiges $C_1$—$C_4$-Alkyl oder Phenyl bedeuten,

b) den erhaltenen Ester der Formel IV

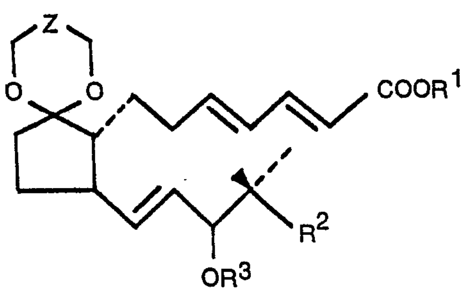

IV

worin $R^1$ die in Formel III, $R^2$ die in Formel I und $R^3$ und Z die in Formel II angegebene Bedeutung haben, durch saure Hydrolyse überführt in eine Verbindung der Formel I, gegebenenfalls die erhaltene Verbindung der Formel I, worin $R^1$ die zur Formel III und $R^2$ die zur Formel I in Anspruch 1 angegebene Bedeutung haben, durch Behandlung mit Basen in eine Verbindung der Formel I, worin $R^1$ Wasserstoff bedeutet und $R^2$ die Zur Formel I in Anspruch 1 angegebene Bedeutung hat, überführt,

gegebenenfalls eine Verbindung der Formel I, worin $R^2$ die zur Formel I in Anspruch 1 angegebene Bedeutung hat und $R^1$ Wasserstoff bedeutet, zu einer Verbindung der Formel I, worin $R^1$ und $R^2$ die zur Formel I in Anspruch 1 angegebenen Bedeutungen haben, wobei $R^1$ jedoch kein kation bedeutet, verestert,

und gegebenenfalls eine Verbindung der Formel I, worin $R^2$ die zur Formel I in Anspruch 1 angegebene Bedeutung hat und $R^1$ Wasserstoff bedeutet, in ein physiologisch verträgliches Metall- oder Aminsalz überführt.

3. Verfahren zur Herstellung von Arzneimitteln, dadurch gekennzeichnet, daß man eine Verbindung der in Anspruch 1 genannten Formel I, gegebenenfalls mit üblichen pharmazeutischen Trägern und/oder Stabilisatoren, in eine therapeutisch geeignete Darreichungsform bringt.

4. Arneimittel, gekennzeichnet, durch einen Gehalt an einer Verbindung der in Anspruch 1 genannten Formel I.

5. Verbindungen der Formel I zur Verwendung als Arzneimittel.

## Claims

1. Compounds of the formula I

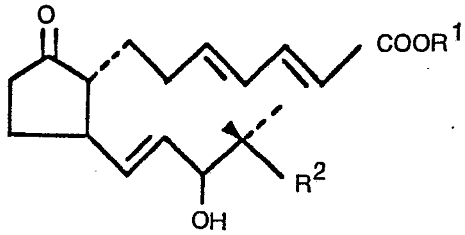

(I)

in which $R^1$ denotes
a) hydrogen or a straight-chain or branched, saturated or unsaturated aliphatic or cycloaliphatic hydrocarbon radical having up to 10 carbon atoms or
b) a physiologically acceptable metal or $NH_4$ion or an an ammonium ion which is derived from a primary, secondary or tertiary amine and
$R^2$ denotes a straight-chain, aliphatic hydrocarbon radical having up to six carbon atoms.

2. A process for the manufacture of compounds of the formula I, which comprises
a) reacting an aldehyde of the formula II

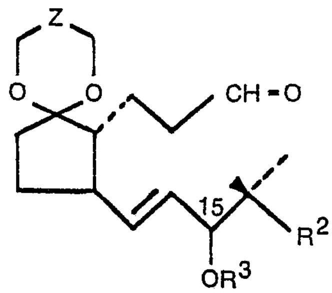

(II)

in which Z denotes a $CH_2$ group, a $—C(CH_3)_2$ group or a single bond, $R^2$ is as defined in formula I and $R^3$ represents an easily detachable protective group, with an ylide of the formula III

$$(R^4)_3P{=}CH{—}CH{=}CH{—}COOR^1 \qquad (III)$$

in which $R^1$ is as defined in formula I but does not represent a cation and in which the substituent $R^4$ can be identical or different and denotes straight-chain $C_1$—$C_4$-alkyl or phenyl, and
b) converting the resulting ester of the formula IV

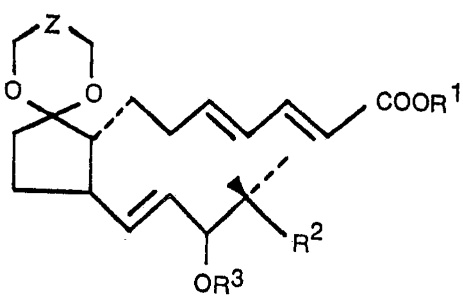

in which $R^1$ is as defined in formula III, $R^2$ is as defined in formula I and $R^3$ and Z are as defined in formula II, by acid hydrolysis to a compound of the formula I and, if desired, converting the resulting compound of the formula I in which $R^1$ is as defined in formula III and $R^2$ is as defined in formula I by treatment with bases to a compound of the formula I in which $R^1$ denotes hydrogen and $R^2$ is as defined in formula I or, if desired, esterifying a compound of the formula I in which $R^2$ is as defined in formula I and $R^1$ denotes hydrogen to a compound of the formula I in which $R^1$ and $R^2$ are as defined in formula I but $R^1$ does not denote a cation and, if desired, converting a compound of the formula I in which $R^2$ is as defined in formula I and $R^1$ denotes hydrogen to a physiologically acceptable metal salt or amine salt.

3. A process for the manufacture of a pharmaceutical composition, which comprises bringing a compound of the formula I as claimed in claim 1, optionally in admixture or conjunction with the

pharmaceutically suitable carriers and/or stabilizers, into a therapeutically suitable dosage unit form.

4. A pharmaceutical composition which comprises an amount of a compound of the formula I as claimed in claim 1.

5. Compounds of the formula I for use as medicaments.

**Revendications**

1. Composés répondant à la formule I

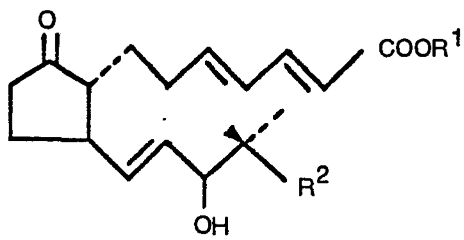

I

dans laquelle $R^1$ représente

a) l'hydrogène ou un radical hydrocarboné aliphatique ou cycloaliphatique, à chaîne droite ou ramifiée, saturé ou insaturé ayant jusqu'à 10 atomes de carbone, ou

b) un ion métallique physiologiquement acceptable ou un ion $NH_4$ ou un ion ammonium provenant d'une amine primaire, secondaire ou tertiaire.

$R^2$ représente un radical hydrocarboné aliphatique, à chaîne droite ayant de 1 à 6 atomes de carbone.

2. Procédé pour la préparation de composés de formule I, caractérisé en ce que:

a) on fait réagir un aldéhyde de formule II

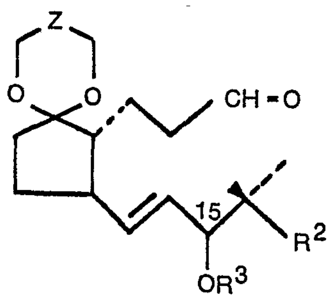

II

où Z représente un groupe $CH_2$, un groupe $—C(CH_3)_2$ ou une simple liaison, $R^2$ a la signification indiquée pour la formule I et $R^3$ représente un groupe protecteur facilement séparable, avec un ylide de formule III

$$(R^4)_3P{=}CH{-}CH{=}CH{-}COOR^1 \qquad \text{III}$$

où $R^1$ a la signification indiquée pour la formule I, mais ne représente pas un cation, et où les substituants $R^4$ peuvent être identiques ou différents et représentent un groupe alkyle en $C_1$—$C_4$ ou phényle.

b) par hydrolyse acide, on convertit l'ester de formule IV obtenu,

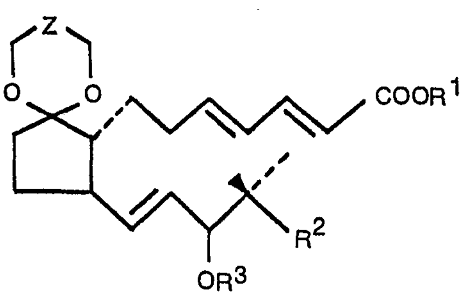

IV

oú $R^1$ a la signification indiquée dans la formule III, $R^2$ a la signification indiquée dans la formule I et $R^3$ et Z ont les significations indiquées dans la formule II, en un composé de formule I; par un traitement avec des bases, on convertit éventuellement le composé de formule I obtenu, où $R^1$ a la signification indiquée pour la formule III et $R^2$ a la signification indiquée pour la formule I dans la revendication 1, en

un composé de formule I où $R^1$ représente l'hydrogène et $R^2$ a la signification indiquée pour la formule I dans la revendication 1, on estérifie éventuellement un composé de formule I, où $R^2$ a la signification indiquée pour la formule I dans la revendication 1 et $R^1$ représente l'hydrogène, en un composé de formule I, où $R^1$ et $R^2$ ont les significations indiquées pour la formule I dans la revendication 1, mais où $R^1$ ne représente pas un cation, et éventuellement on convertit un composé de formule I où $R^2$ a la signification indiquée pour la formule I dans la revendication 1, et $R^1$ représente l'hydrogène, en un sel de métal ou d'amine physiologiquement acceptable.

3. procédé pour la fabrication de médicaments, caractérisé en ce qu'on met sous une forme d'administration thérapeutiquement appropriée un composé de formule I selon la revendication 1, éventuellement avec des véhicules et/ou des stabilisants pharmaceutiques usuels.

4. Médicaments caractérisés par une teneur en un composé de formule I selon la revendication 1.

5. Composés de formule I à utiliser comme médicaments.